# EUROPÄISCHE PATENTSCHRIFT

(11) **EP 4 340 776 B1**
(45) Veröffentlichungstag und Bekanntmachung des Hinweises auf die Patenterteilung: **18.02.2026**
(21) Anmeldenummer: 22729535.9
(22) Anmeldetag: 16.05.2022
(51) Int. Cl.: A61F 2/16

(54) **INJEKTOR FÜR EINE INTRAOKULARLINSE**
INJECTOR FOR AN INTRAOCULAR LENS
INJECTEUR POUR LENTILLE INTRAOCULAIRE

(30) Priorität: 17.05.2021 DE 102021112682
(43) Veröffentlichungstag der Anmeldung: 27.03.2024
(73) Patentinhaber: Iolution GmbH, 22761 Hamburg (DE)
(72) Erfinder: MAROSCHECK, Christoph, 22761 Hamburg (DE); ZEHNDER, Thomas, 6300 Zug (AT); GUTIERREZ, Maximiliano, 22765 Hamburg (DE)
(74) Vertreter: Friderichs, Gunther
(86) Internationale Anmeldenummer: PCT/EP2022/063167
(87) Internationale Veröffentlichungsnummer: WO 2022/243233

(56) Entgegenhaltungen:
- EP-A1- 3 476 375
- EP-A1- 3 738 556
- WO-A1-2011/126144

## Beschreibung

### Gebiet der Erfindung

Die Erfindung betrifft einen Injektor für eine Intraokularlinse. Insbesondere betrifft die Erfindung einen als Einwegsystem ausgebildeten Injektor, mit welchem eine Intraokularlinse im Auge des Patienten abgegeben und positioniert werden kann.

### Hintergrund der Erfindung

Beim Einsetzen von Intraokularlinsen als Ersatz für die natürliche Linse des Auges, wie dies insbesondere bei Behandlung des Grauen Star vorgenommen wird, haben sich Injektoren etabliert.

Ein derartiger Injektor umfasst einen Kolben, über den die Linse durch eine Kanüle in das Auge des Patienten überführt wird.

Bei derartigen Injektoren ist insbesondere von Vorteil, dass die Linse auch in einem gefalteten Zustand in das Auge transportiert werden kann, so dass der für die Kanüle erforderliche Kanal einen kleineren Querschnitt hat als der Kanal, welcher zum Einbringen einer nicht gefalteten Linse in das Auge geschnitten werden muss.

Ist die Linse an der Spitze der Kanüle angekommen, so ist der dann noch erforderliche Vorschubweg recht gering. Insbesondere beträgt dieser auch bei Linsen mit einer Haptik meist weniger als 12 mm.

Zudem kann sich durch die verjüngende Kanüle die zum Vortrieb erforderliche Kraft zunächst erhöhen, um sodann sprunghaft abzunehmen, wenn die Linse teilweise die Kanüle verlassen hat.

Somit kann es schwierig sein, während des Drückens auf den Kolben die Position exakt einzuhalten.

Die Patentschrift EP 2 150 204 B1 sieht daher vor, dass der Kolben ein Gewinde aufweist, über welches eine Drehbewegung am Griff des Kolbens in eine Linearbewegung desselben umgesetzt wird.

Der der Benutzer für den gesamten Abgabevorgang so lange drehen müsste, sieht vorstehend genannte Patentschrift vor, dass über eine Kulissenführung mit Sperrkugeln das Gewinde des Kolbens nur dann in Eingriff ist, wenn dies vom Benutzer gewünscht ist.

Von Nachteil bei dem Injektor gemäß der Lehre dieses Dokuments ist, dass der Benutzer zunächst den Ring mit der Sperrkulisse betätigen muss, um die Kugeln mit dem Gewinde in Eingriff zu bringen. Sodann muss der Benutzer umgreifen, um den Injektor am Gehäuse zu halten und anschließend durch Drehung am Griff der Kolbenstange den Kolben weiter vorzuschieben.

Das Umgreifen kann Positionierungsfehler zur Folge haben. Des Weiteren ist es so kaum möglich, mehrfach zwischen der Verwendung des Drehelements und einem Vorschub durch Drücken auf den Griff zu wechseln. Ein Zurückwechseln auf manuelles Vorschieben kann aber situationsbedingt sinnvoll sein, insbesondere dann, wenn die Linse schon teilweise herausgekommen und optimal positioniert ist. Auch gibt es Anwender, die tendenziell lieber durch Verwendung eines Drehelements oder durch Drücken die Linse vorschieben.

Weiter ist der Injektor gemäß vorstehend beschriebenem Stand der Technik recht aufwendig ausgestaltet. Insbesondere werden für eine Sperrkulisse in der Regel Metallkugeln verwendet, so dass der Injektor teils aus Kunststoff, teils aus Metall ausgebildet ist, was wiederum dessen Entsorgung oder Recyclebarkeit erschwert.

Das Dokument EP 3 476 375 A1 zeigt einen Injektor, bei dem ein Drehelement drehbar auf dem Injektorgehäuse gelagert ist.

### Aufgabe der Erfindung

Der Erfindung liegt demgegenüber die Aufgabe zugrunde, die genannten Nachteile des Standes der Technik zumindest zu reduzieren.

Es ist insbesondere eine Aufgabe der Erfindung, einen Injektor zur Abgabe einer Intraokularlinse bereitzustellen, welcher sich sowohl einfach herstellen lässt als auch eine einfache und sichere Benutzung ermöglicht.

### Zusammenfassung der Erfindung

Die Aufgabe der Erfindung wird bereits durch einen Injektor für eine Intraokularlinse nach Anspruch 1 gelöst.

Bevorzugte Ausführungsformen und Weiterbildungen der Erfindung sind dem Gegenstand der abhängigen Ansprüche, der Beschreibung sowie den Zeichnungen zu entnehmen.

Die Erfindung betrifft einen Injektor für eine Intraokularlinse, welche ein Gehäuse mit einer Kanüle umfasst, einen Aufnahmeraum für die Intraokularlinse sowie einen im Gehäuse verschiebbaren Kolben.

Der Aufnahmeraum kann insbesondere auch einstückig mit der Kanüle als ein einziges Bauteil ausgebildet sein, welches z.B. mit einem Hauptgehäuse des Injektors verbunden ist. Gemäß einer Ausführungsform kann auf den Aufnahmeraum ein Magazin aufgesetzt werden, welches die Intraokularlinse enthält. Über ein Faltelement kann die Linse sodann in den Aufnahmeraum transferiert werden, wobei sie sich gleichzeitig faltet.

Durch Vorschub des Kolbens kann sodann die Linse zur Spitze der Kanüle transferiert und ins Auge des Patienten abgegeben werden.

Gemäß der Erfindung umfasst der Injektor ein Drehelement zum Verschieben des Kolbens, wobei das Drehelement sowohl drehbar als auch axial und linear, ohne sich zu drehen, verschiebbar auf dem Kolben gelagert ist.

Zur drehbaren Lagerung kann das Drehelement insbesondere auf einer Kolbenstange des Kolbens geführt sein. Insbesondere können Drehelement und Kolbenstange eine Übermaßpassung bilden. Das Drehelement lässt sich so gegen die Reibung der Übermaßpassung drehen und sitzt ohne Spiel auf der Kolbenstange.

Der Kolben ist wiederum gegenüber dem Drehelement in distaler Richtung axial verschiebbar.

Neben einem Vorschub über das Drehelement ist also vorgesehen, dass der Kolben auch von Hand durch Drücken auf einen Griff am proximalen Ende des Kolbens vorgeschoben werden kann.

Das Drehelement kann insbesondere in einer Ausgangsposition derart von einem Hauptgehäuse des Injektors entfernt sein, dass ein Gewinde des Drehelements nicht im Eingriff mit einer korrespondierenden Gewindestruktur des Hauptgehäuses des Injektors ist. So kann der Benutzer den Kolben erst durch Drücken auf ein proximales Ende des Kolbens nach vorne schieben.

Sodann kommt das Drehelement an einer Aufnahme zur Anlage.

Nunmehr kann der weitere Vorschub über das Drehelement vorgenommen werden, indem ein Gewinde des Drehelements in Eingriff kommt, so dass nunmehr der Kolben auch über das Drehelement nach vorne geschoben werden kann.

Gemäß einer Ausführungsform der Erfindung ist vorgesehen, dass der Kolben, wenn das Drehelement bereits an der Aufnahme im Eingriff ist, auch weiter durch Drücken auf das proximale Ende des Kolbens vorgeschoben werden kann.

Der Benutzer kann also so je nach Belieben zwischen einem Vorschub durch Drücken auf den Kolben oder einem Vorschub durch Drehen des Drehelements wechseln.

Die Aufnahme für das Drehelement befindet sich vorzugsweise angrenzend zu einem Griff, insbesondere einem Flügelgriff.

Der Griff kann als Gegenlager für den Benutzer dienen, wenn dieser den Kolben durch Drücken vorschiebt.

Zur Betätigung des Drehelements muss der Benutzer nur die Finger von einer Hand an das Drehelement setzen, wobei während des Drehens die am proximalen Ende befindliche Hand des Benutzers zur Führung verwendet werden kann.

Entscheidet sich der Benutzer nunmehr, die Linse nicht vollständig durch Drehung des Drehelements aus der Kanüle auszupressen, kann das Gehäuse mit dem Drehelement, dessen Gewinde nunmehr im Eingriff ist, nur noch als Gegenhalter dienen, um einen restlichen Auspressvorgang durch Drücken auf den Kolben zu beenden.

Durch die Erfindung wird eine einfache Ausgestaltung eines Injektors aus wenigen Teilen ermöglicht. Insbesondere kann auf Metallteile zumindest weitgehend, vorzugsweise vollständig, verzichtet werden.

Weiter wird, wie bereits vorstehend ausgeführt, eine besonders einfache Bedienung und vorzugsweise auch das einfache Wechseln zwischen Vorschub durch Drehen und Vorschub durch Drücken ermöglicht.

Der Injektor ist erfindungsgemäß derart ausgebildet, dass das Gewinde des Drehelements mit der Aufnahme in Eingriff kommt, wenn sich die Intraokularlinse bereits in der Kanüle befindet.

Das Überführen vom Aufnahmeraum in die Kanüle, welche sich vorzugsweise konisch nach vorne verjüngt, erfolgt also vorzugsweise allein durch Drücken auf das distale Ende des Kolbens.

Insbesondere ist vorgesehen, dass das Gewinde mit der Aufnahme in Eingriff kommt, wenn sich die Intraokularlinse unmittelbar vor der Spitze der Kanüle befindet. Es ist also vorgesehen, dass lediglich der letzte Teil des Abgabevorgangs, nämlich das Auspressen der Linse aus der Spitze der Kanüle, über das Drehelement vornehmbar ist.

Bei einer Ausführungsform der Erfindung ist das Drehelement auf dem Kolben durch einen Sicherungsring gegen eine Verschiebung in distaler Richtung gesichert.

Der Sicherungsring kann insbesondere einen Schlitz aufweisen und so auseinandergebogen und auf die Kolbenstange aufgesetzt werden.

Dies ermöglicht eine einfache Herstellung und Montage.

Weiter wirkt der Sicherungsring als Anschlag, welcher den Kolben beim Drehen am Drehelement nach vorne mitnimmt.

Es versteht sich, dass bei Verwendung des Drehelements zum Vorschub des Kolbens und anschließendem weiteren Vorschub durch Drücken auf den Kolben das Drehelement erst dann wieder an dem Sicherungsring zum Anschlag kommt, wenn dieser entsprechend weiter vorgedreht wird. Eine derartige Situation wird aber bei Benutzung des erfindungsgemäßen Injektors eher selten auftreten. Wenn sich der Benutzer entscheidet, von einem Vorschub über eine Drehung wieder in einen Vorschub durch Drücken auf den Kolben überzugehen, ist die Linse in der Regel bereits wunschgemäß positioniert und wird sodann durch ein finales einmaliges Drücken auf den Kolben nur noch ausgepresst, um sich dann im Auge zu entfalten.

Bei einer Ausführungsform der Erfindung ist elastischer Stopper zwischen Kolben und Gehäuse vorhanden, welcher als Anschlag für den Kolben in seiner Endposition wirksam ist. Der elastische Stopper kann z.B. in das Gehäuse eingelegt oder auf den Kolben aufgesteckt sein.

In der Endposition wird so ein "weicher" Anschlag bereitgestellt, welcher das Erreichen der Endposition gut haptisch wahrnehmbar macht.

Bei einer Ausführungsform der Erfindung ist der Kolben in einem Hauptgehäuse des Injektors in einer Ausgangsposition verrastet.

Insbesondere kann der Kolben einen Rasthaken umfassen, welcher bei einer Montage in einer Ausnehmung des Hauptgehäuses verrastet. Der Kolben kann nunmehr nicht mehr zurückgezogen werden.

Wird der Kolben dagegen nunmehr in distaler Richtung vorgeschoben, so federt der Rasthaken ein und erlaubt einen weiteren Vorschub des Kolbens.

Die Kolbenstange kann einen kreuzförmigen Querschnitt aufweisen.

Dies erleichtert eine Lagerung der Kolbenstange im Gehäuse.

Weiter kann sich die Kolbenstange stufenweise in proximaler Richtung verdicken. Insbesondere kann ein vorderer Teil der Kolbenstange einen geringeren Außendurchmesser als ein hinterer Teil der Kolbenstange aufweisen.

Der hintere Teil der Kolbenstange ist so robuster und kann in einem Hauptgehäuse geführt sein, wohingegen der vordere Teil aus dem Hauptgehäuse in den Aufnahmeraum bzw. bei weiterem Vorschieben in die Kanüle ragt.

Die Aufnahme im Gehäuse kann ein segmentiertes Außengewinde für das Drehelement aufweisen.

Dies erleichtert das Ineingriffkommen des Innengewindes des Drehelements.

Unter einem Gewinde im Sinne der Erfindung werden sämtliche in Eingriff befindlichen Bauteile verstanden, über die eine Drehbewegung des Drehelements in eine axiale Linearbewegung des Kolbens umsetzbar ist.

Insbesondere müssen die Eingriffelemente an der Aufnahme am Hauptgehäuse nicht als Gewindezüge mit einer Steigung ausgebildet sein, sondern es genügt die Anordnung von Rippen, welche mit dem Innengewinde des Drehelements in Eingriff kommen.

Bei einer Ausführungsform der Erfindung kommt das Drehelement nach einem Vorschub des Kolbens von mehr als 10 mm und/oder von weniger als 30 mm an der Aufnahme zur Anlage. Der Kolben ist vorzugsweise um mehr als 5 mm, besonders bevorzugt mehr als 8 mm und/oder weniger als 15 mm, besonders bevorzugt weniger als 12 mm allein durch Drehung am Drehelement verschiebbar.

Das Drehelement kann ein Innengewinde mit einer Steigung zwischen 1,5 und 7 mm aufweisen.

Weiter kann das Drehelement einen Außendurchmesser von 18 bis 28 mm aufweisen.

Die Offenbarung betrifft des Weiteren ein Verfahren zum Abgeben einer Intraokularlinse aus einem Injektor. Zur Ausführung des Verfahrens kann insbesondere der vorstehend beschriebene Injektor verwendet werden.

Das Verfahren kann insbesondere zu Testzwecken, wie einer Funktionsprüfung des Injektors, durchgeführt werden.

Gemäß des Verfahrens ist die Linse zunächst in einem Aufnahmeraum des Injektors in einem gefalteten Zustand angeordnet.

Insbesondere kann die Linse durch das Schließen eines Deckels mit einem Faltelement gefaltet werden.

Sodann wird durch Drücken auf den Griff eines axial verschiebbaren Kolbens die Linse aus dem Aufnahmeraum in eine Kanüle überführt. Die Kanüle verjüngt sich insbesondere zu einem distalen Ende hin.

Erfindungsgemäß kommt, während sich die Intraokularlinse in der Kanüle befindet, ein auf einer Kolbenstange gelagertes Drehelement an einer Aufnahme für das Drehelement zur Anlage.

Sodann ist der Kolben sowohl durch weiteres Drücken auf den Griff als auch durch Drehen am Drehelement vorschiebbar, um die Intraokularlinse aus der Kanüle abzugeben. Wesentlich für die Erfindung ist, dass zunächst, um die Linse aus der Aufnahme in die Kanüle zu überführen, dies ausschließlich durch Drücken auf den Kolben erfolgt.

Dies hat den Vorteil, dass dieser Schritt relativ schnell geht.

Insbesondere kann dieser Verfahrensschritt ausgeführt werden, wenn die Kanüle noch nicht in das Auge des Benutzers eingestochen ist.

Dieser Verfahrensschritt kann insbesondere von der Assistenz des Augenchirurgen durchgeführt werden.

Sofern Drehelement und Kolbenstange eine Übermaßpassung bilden, lässt sich durch einen haptisch wahrnehmbaren Widerstand bemerken, dass nunmehr das Drehelement an der Aufnahme zur Anlage gekommen ist.

Die Assistenz kann nunmehr den vorbereiteten Injektor dem Augenchirurgen überreichen und dieser hat sodann die volle Freiheit, ob er die Linse lieber durch Drücken auf den Kolben oder unter Verwendung des Drehelements ins Auge presst.

### Kurzbeschreibung der Zeichnungen

Der Gegenstand der Erfindung soll im Folgenden bezugnehmend auf ein Ausführungsbeispiel anhand der Zeichnungen Fig. 1 bis Fig. 9 näher erläutert werden.
Fig. 1 bis Fig. 4 sind isometrische Ansichten eines Ausführungsbeispiels eines Injektors in einen Betriebszuständen. Dargestellt ist also, wie der Injektor verwendet wird, um eine Intraokularlinse abzugeben.
Fig. 5 ist ein Längsschnitt des Injektors.
Fig. 6 ist eine isometrische Ansicht des Kolbens.
Fig. 7 ist eine perspektivische Ansicht des Hauptgehäuses auf dessen proximales Ende.
Fig. 8 und Fig 9 sind Detailansichten eines Längsschnitts im Bereich des Drehelements in verschiedenen Betriebszuständen des Injektors.

### Detaillierte Beschreibung der Zeichnungen

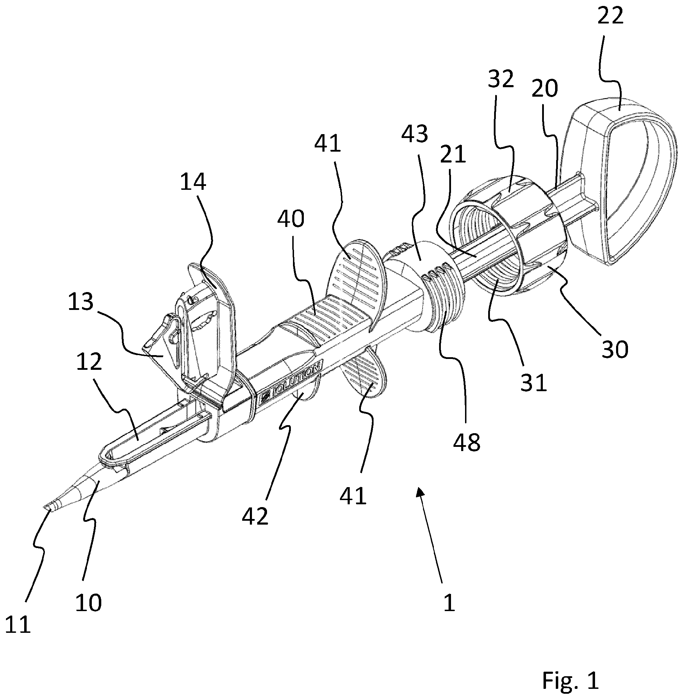

Fig. 1 ist eine isometrische Ansicht eines Ausführungsbeispiels eines erfindungsgemäßen Injektors 1.

Der Injektor 1 umfasst eine Kanüle 10 mit einer Spitze 11, über die eine Intraokularlinse (nicht dargestellt) ins Auge eines Patienten (nicht dargestellt) abgebbar ist.

Die Intraokularlinse wird hierzu in den Aufnahmeraum 12 eingesetzt und sodann mittels des Kolbens 20 aus der Kanüle 10 gepresst.

In diesem Ausführungsbeispiel kann eine in einem Magazin (nicht dargestellt) befindliche Intraokularlinse in den Aufnahmeraum 12 eingebracht werden, indem das Magazin zunächst auf den Injektor, insbesondere auf das Vorderteil mit der Kanüle 10 und dem Aufnahmeraum aufgesetzt wird.

Dargestellt ist hier der noch nicht fertig montierte Zustand, in welchem das bewegliche Faltelement 13 noch nicht in den Träger 16 eingesetzt ist.

Im montierten Zustand sitzt ein Stift 18 des Faltelements 13 in der Führungskulisse 17 des Trägers. So wird eine definierte Beweglichkeit des Faltelements 13 während des Auspressens der Linse ermöglicht.

Vor dem Auspressen der Linse wird der fertig montierte Deckel 14 mit dem Faltelement geschlossen und das mit dem Deckel 14 verbundene Faltelement 13 transferiert die Intraokularlinse in den Aufnahmeraum 12, in welchem diese sogleich gefaltet wird. Meist wird vor dem Falten das Faltelement 13 in dem Deckel 14 vorgeschoben und dann kann der Deckel 14 geschlossen werden.

Auch in den folgenden Zeichnungen ist nicht dargestellt, wie der Deckel mit dem Faltelement geschlossen wird.

Das Grundprinzip der Erfindung lässt sich aber auf alle Arten von Injektoren für Intraokularlinsen übertragen, insbesondere solche, die bereits mit einer Linse vorgeladen sind. Der Injektor 1 umfasst ferner das Hauptgehäuse 40, in welchem der Kolben 20 geführt ist. Der Kolben 20 umfasst die in das Hauptgehäuse 40 ragende Kolbenstange 21, welche am proximalen Ende den Griff 22 umfasst.

In Fig. 1 ist die Ausgangsposition des Injektors 1 dargestellt, also der Betriebszustand, bevor der Injektor 1 verwendet wird.

In diesem Betriebszustand ist das Drehelement 30 drehbar auf der Kolbenstange 21 gelagert und noch von der Aufnahme 43 für das Drehelement 30 beabstandet.

Das Drehelement 30 umfasst ein Innengewinde 31.

Das Drehelement 30 ist in diesem Ausführungsbeispiel im Wesentlichen kreiszylinderförmig ausgestaltet und umfasst eine Profilierung 32, um dieses besser greifen zu können.

Der Benutzer kann nunmehr den Kolben 20 vorschieben, bis das Drehelement 30 an der Aufnahme 43 zur Anlage kommt.

Die Aufnahme 43 umfasst ein Gewinde 48, welches in diesem Ausführungsbeispiel segmentiert ausgebildet ist, um das Entformen des Gewindes 31 des Drehelements 30 in einem Spitzgusswerkzeug zu erleichtern.

Angrenzend zur Aufnahme befindet sich in distaler Richtung ein Flügelgriff 41, an welchem der Benutzer den Injektor 1 beispielsweise mit Zeigefinger und Ringfinger halten kann und welcher so ein Gegenlager bildet, wenn der Benutzer den Kolben 20 am Griff 22 vorschiebt. Insbesondere kann so der Injektor auch einhändig verwendet werden, indem der Griff 22 mit der Innenfläche der Hand vorgeschoben wird, während zwei Finger den Injektor 1 am Flügelgriff 41 halten.

Auf der Unterseite befindet sich in distaler Richtung nach dem Flügelgriff 41 ein weiterer Griff 42, welcher optional verwendet werden kann, etwa um eine Feinjustierung der Position der Spitze 11 der Kanüle 10 vorzunehmen oder um als Gegenhalter für den Mittelfinger zu dienen.

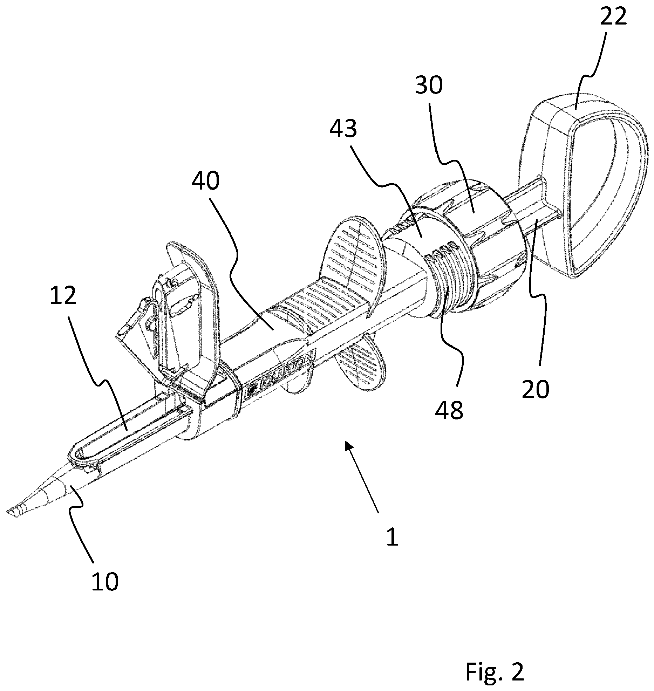

Wie in Fig. 2 dargestellt, wird der Kolben 20 zunächst so weit vorgeschoben, bis das Drehelement 30 an der Aufnahme 43, welche in diesem Ausführungsbeispiel das proximale Ende des Hauptgehäuses 40 bildet, zur Anlage kommt.

Die Intraokularlinse ist nunmehr bereits aus dem Aufnahmeraum 12 heraus in die Kanüle 10 transferiert. Es versteht sich, dass der Deckel 14 dabei geschlossen ist (nicht dargestellt). Das Drehelement 30 ist nun zwar weiter in proximaler Richtung gegenüber dem Kolben 20 verschiebbar. Aufgrund einer Übermaßpassung zwischen Drehelement 30 und Kolben 20 spürt der Benutzer aber einen Widerstand, der signalisiert, dass nunmehr die Intraokularlinse zum Einpressen in das Auge bereit ist.

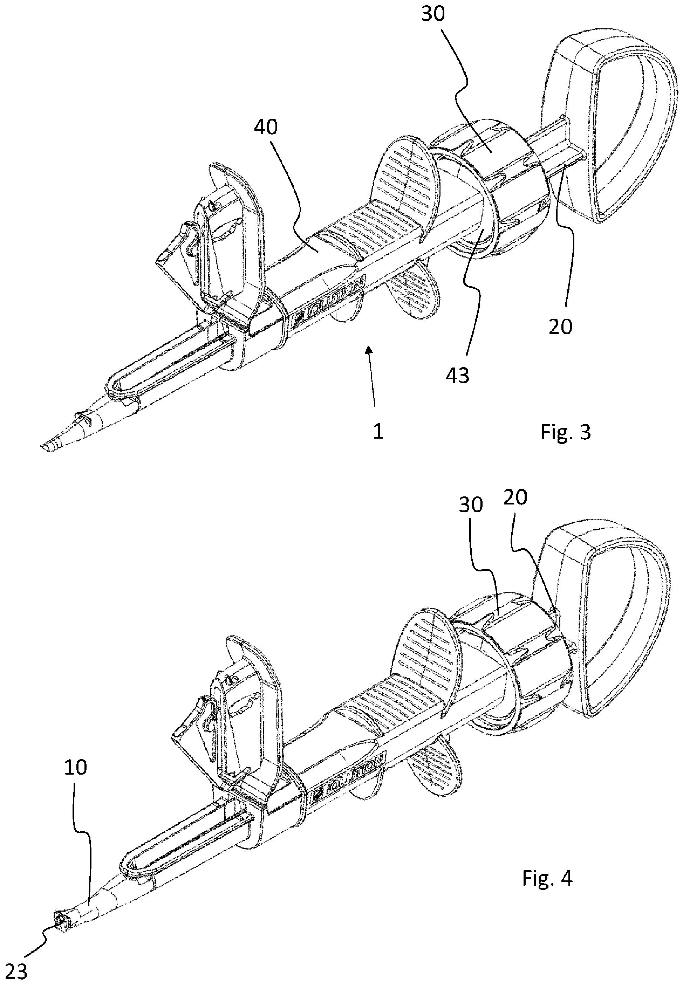

Der Benutzer kann nunmehr, wie im Folgenden gemäß Fig. 3 und Fig. 4 dargestellt, am Drehelement 30 drehen, um den Kolben 20 weiter vorzuschieben.

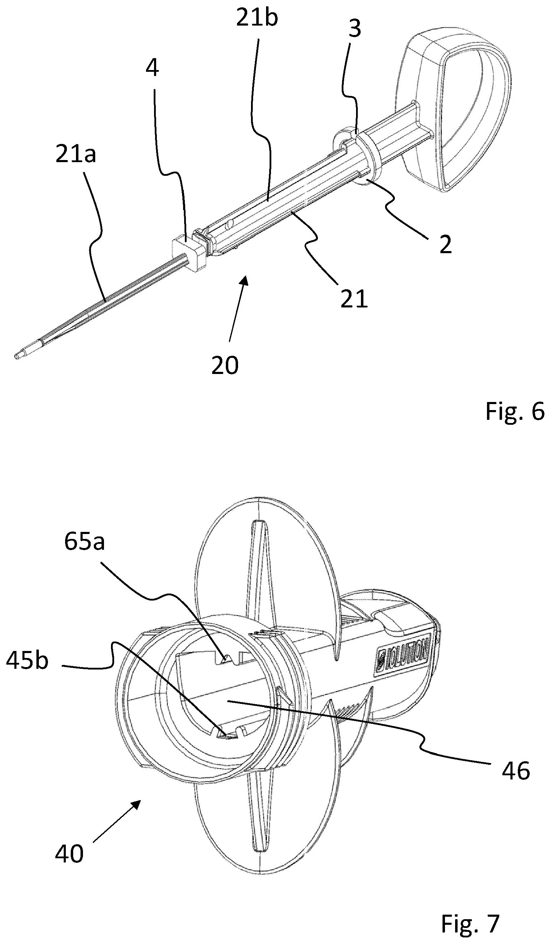

Alternativ kann der Benutzer auch weiter den Griff 22 nach vorne schieben und so die Intraokularlinse aus der Kanüle 10 herausdrücken, ohne das Drehelement 30 zu verwenden. Verwendet der Benutzer das Drehelement, so kann er, wie in Fig. 4 dargestellt, das Drehelement 30 so lange drehen, bis der Kolben 20 in seiner Endposition zur Anlage kommt. Vorzugsweise kommt das Drehelement 43 in der Endposition nicht an der Aufnahme 43 zu Anlage, sondern die Endposition wird allein durch einen elastischen Stopper (siehe 4 in Fig. 6) definiert.

Die Intraokularlinse ist nunmehr in das Auge des Patienten transferiert.

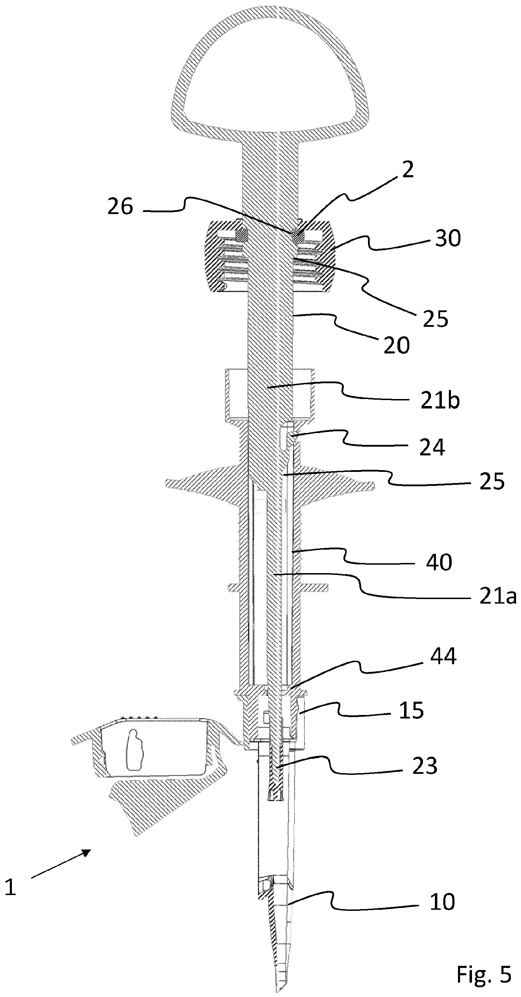

Fig. 5 ist ein Längsschnitt des Injektors in seinem Ausgangszustand.

Das vordere Gehäuseteil mit der Kanüle 10 umfasst in diesem Ausführungsbeispiel eine Nut 15, mit der es gegenüber dem Hauptgehäuse 40 gesichert ist.

Die Kanüle 10 kann also auf das Hauptgehäuse 40 aufgerastet werden.

Die Kolbenstange 21 ist in mehrere Abschnitte unterteilt.

Ein vorderer Abschnitt 21a hat einen kleineren Durchmesser als der dahinterliegende Abschnitt 21b.

Der Abschnitt 21a dient der Aufnahme der Spitze 23 aus elastischem Material.

Im distalen vorderen Bereich des Hauptgehäuses 40 befindet sich eine Führung 44 für den vorderen Teil 21a der Kolbenstange 21.

Ein dahinterliegender Teil 21b der Kolbenstange wird im Hauptgehäuse 40 geführt. Die Kolbenstange 21 umfasst in diesem Bereich einen Rasthaken 24, welcher bei der Montage im Hauptgehäuse 40 verrastet wird und sodann nur noch eine Bewegung des Kolbens 20 in distaler Richtung zulässt.

Der hintere Teil 21b der Kolbenstange 21 umfasst im Bereich des Drehelements 30 zunächst eine Stufe 25.

Hiernach folgt eine Nut 26, in welcher der Sicherungsring 2 sitzt. Der Sicherungsring 2 bildet einen Anschlag für das Drehelement 30 in distaler Richtung.

Im Ausgangszustand befindet sich das Drehelement 30 an diesem Anschlag.

Um einen Anschlag in der Endposition des Kolbens 20 bereitzustellen, ist in dieser Ausführungsform ein elastischer Stopper 4 auf den Kolben 20 aufgeschoben.

Die vordere Führung 44 bildet eine Wand, an welcher der elastische Stopper 4 in der Endposition zur Anlage kommt und so den Vorschub durch ein federndes Stoppen beendet. Gemäß einer anderen Ausführungsform kann der elastische Stopper auch in das Hauptgehäuse eingesetzt sein, statt auf den Kolben aufgeschoben zu sein (nicht dargestellt).

Fig. 6 ist eine perspektivische Ansicht des Kolbens 20 nebst Sicherungsring 2.

Der Sicherungsring 2 hat einen Schlitz 3, über den dieser aufgeweitet und in die Nut der Kolbenstange 21 eingesetzt werden kann.

Sowohl der vordere Teil der Kolbenstange 21a als auch der hintere Teil 21b der Kolbenstange 21 hat einen kreuzförmigen Querschnitt, um in einer entsprechenden Führung des Gehäuses des Injektors zu gleiten.

Weiter sitzt der elastische Stopper 4 auf dem vorderen Teil 21a der Kolbenstange 21.

Dieser dient einem federnden Kontakt, wenn der Kolben 20 seine Endposition erreicht.

Fig. 7 ist eine perspektivische Ansicht auf das proximale Ende des Hauptgehäuses 40.

Das Hauptgehäuse umfasst für die Kolbenstange, genauer gesagt für den hinteren Teil (21b) der Kolbenstange einen Kanal 46.

In den Kanal 46 ragt eine obere Führungsschiene 45a sowie eine untere Führungsschiene 45b. Über diese Führungsschienen wird die Kolbenstange im Hauptgehäuse 40 geführt.

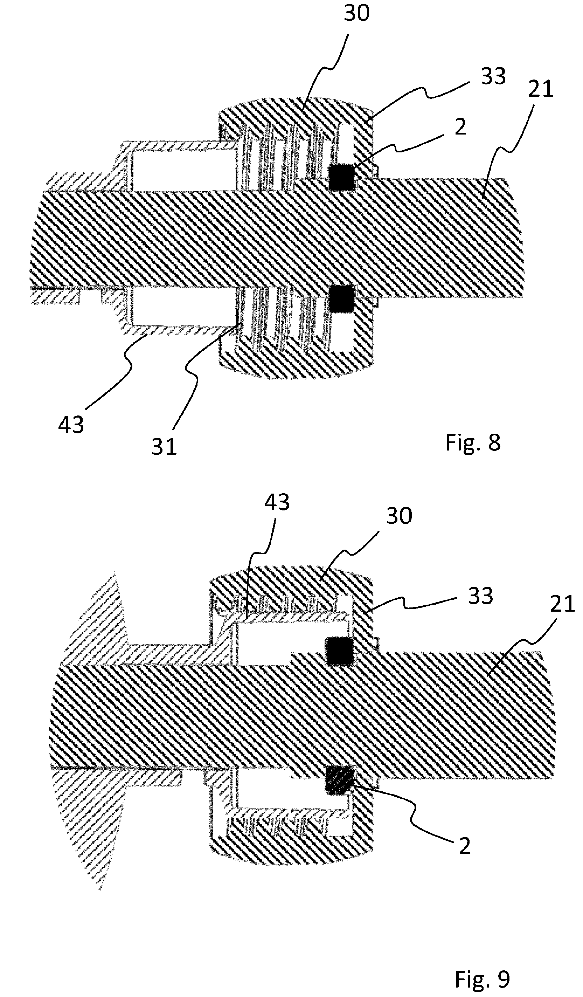

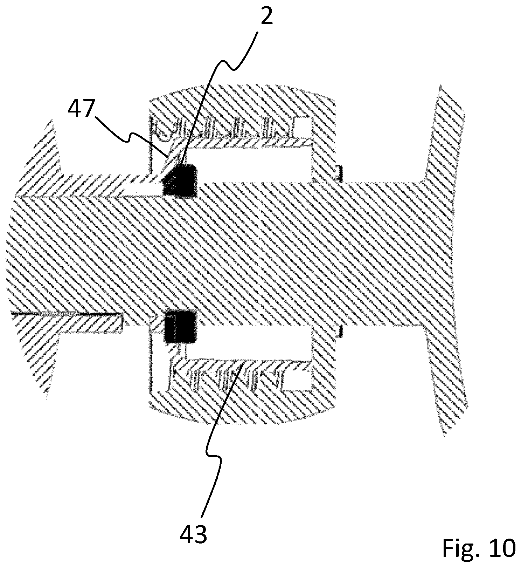

Fig. 8 zeigt in einer Detaildarstellung die Position des Drehelements, wenn dieses an der Aufnahme 43 zur Anlage kommt. Diese Position entspricht der Darstellung gemäß Fig. 2. Das Drehelement 30, welches mit der Wand 33 drehbar auf der Kolbenstange 21 gelagert ist, wird zusammen mit der Kolbenstange 21 nach vorne, also in distaler Richtung bewegt, bis das Gewinde 31 am Gewinde der Aufnahme 43 zur Anlage kommt.

Drückt nun der Benutzer weiter auf das proximale Ende des Kolbens 20, so bewegt sich die Kolbenstange 21 weiter nach vorne. Der Sicherungsring 2 wirkt neben dem Drehelement 30 nur als Anschlag gegenüber einer Bewegung der Kolbenstange 21 in proximaler Richtung (nicht dargestellt).

Dreht nunmehr, wie in Fig. 9 dargestellt, der Benutzer an dem Drehelement 30, um die Intraokularlinse möglichst langsam auszupressen, so schiebt die Wand 33, welche am Sicherungsring 2 anliegt, die Kolbenstange 21 nach vorne, bis das Drehelement 30 in die Endposition kommt.

Eine weitere Drehung des Drehelements 30 ist nun nicht mehr möglich, da ein weiterer Vorschub des Kolbens durch den Stopper blockiert ist. Der Sicherungsring 2 kommt also an der Wand 47 nicht zur Anlage.

Durch die Erfindung konnte ein einfach aufgebauter, aber sehr gut bedienbarer Injektor für Intraokularlinsen bereitgestellt werden. Dieser ermöglicht insbesondere die optionale Verwendung eines Drehelements, während die Intraokularlinse die Kanüle des Injektors verlässt.

### Bezugszeichenliste

- 1: Injektor
- 2: Sicherungsring
- 3: Schlitz
- 4: elastischer Stopper
- 10: Kanüle
- 11: Spitze
- 12: Linsenaufnahme
- 13: Faltelement
- 14: Deckel
- 15: Nut
- 16: Träger
- 17: Führungskulisse
- 18: Stift
- 20: Kolben
- 21: Kolbenstange
- 22: Griff
- 23: Spitze
- 24: Rasthaken
- 25: Stufe
- 26: Nut
- 30: Drehelement
- 31: Innengewinde
- 32: Profilierung
- 40: Hauptgehäuse
- 41: Flügelgriff
- 42: vorderer Griff
- 43: Aufnahme für das Drehelement
- 44: Führung für 21a
- 45a: obere Führungsschiene
- 45b: untere Führungsschiene
- 46: Kanal für 21b
- 47: Wand
- 48: Gewinde

## Patentansprüche

1. Injektor (1) für eine Intraokularlinse, umfassend ein Gehäuse mit einer Kanüle (10), einen Aufnahmeraum (12) für die Intraokularlinse, sowie einen im Gehäuse verschiebbaren Kolben (20), der dazu ausgebildet ist, die Intraokularlinse aus dem Aufnahmeraum (12) durch die Kanüle (10) zu bewegen, wobei der Injektor (1) ein Drehelement (30) zum Verschieben des Kolben aufweist (20), **dadurch gekennzeichnet dass** das Drehelement (30) drehbar und axial verschiebbar auf dem Kolben (20) gelagert ist, wobei in einer Ausgangsposition das Drehelement (30) von einer an einem Hauptgehäuse (40) angeordneten Aufnahme (43) beabstandet ist, wobei das Drehelement (30) mit dem Kolben (20) verschiebbar ist, bis das Drehelement (30) mit der Aufnahme (43) in Kontakt kommt und sodann mit einem Gewinde (31) mit der Aufnahme (43) in Eingriff kommt.

2. Injektor (1) nach dem vorstehenden Anspruch, **dadurch gekennzeichnet, dass** der Injektor (1) derart ausgebildet ist, dass das Gewinde (32) mit der Aufnahme in Eingriff kommt, wenn sich die Intraokularlinse in der Kanüle (10) befindet, insbesondere wenn sich die Intraokularlinse unmittelbar vor einer Spitze (11) der Kanüle (10) befindet.

3. Injektor (1) nach dem vorstehenden Anspruch, **dadurch gekennzeichnet, dass** die Intraokularlinse durch eine Drehung am Drehelement (30) aus der Kanüle (10) herausschiebbar ist.

4. Injektor (1) nach einem der vorstehenden Ansprüche, **dadurch gekennzeichnet, dass** das Drehelement (30) auf dem Kolben (20) durch einen Sicherungsring (2) gegen eine Verschiebung in distaler Richtung gesichert ist.

5. Injektor (1) nach einem der vorstehenden Ansprüche, **dadurch gekennzeichnet, dass** ein Vorschub des Kolbens (20) in einer Endposition durch einen elastischen Stopper (4) blockiert ist.

6. Injektor (1) nach einem der vorstehenden Ansprüche, **dadurch gekennzeichnet, dass** der Kolben (20), nachdem das Drehelement (30) zur Endposition gelangt ist, weiter über einen Griff (22) vorschiebbar ist, insbesondere über eine Strecke von 1 bis 8 mm.

7. Injektor (1) nach einem der vorstehenden Ansprüche, **dadurch gekennzeichnet, dass** die Intraokularlinse gefaltet in dem Aufnahmeraum (12) angeordnet ist,
und/oder dass der Kolben (20) in einem Hauptgehäuse (40) verrastet ist,
und/oder dass das Drehelement (30) im Wesentlichen als Kreiszylinder mit einer proximalen Wand ausgebildet ist, welche eine zentrale Öffnung aufweist, die im Zusammenwirken mit einer Kolbenstange (21) ein Drehlager bildet,
und/oder dass die Kolbenstange (21) einen kreuzförmigen Querschnitt aufweist, und/oder dass die Aufnahme (43) für das Drehelement (30) ein segmentiertes Außengewinde (48) aufweist,
und/oder dass das Drehelement (30) nach einem Vorschub des Kolbens (20) von mehr als 10 mm und/oder von weniger als 30 mm an der Aufnahme (43) zur Anlage kommt, und/oder dass der Kolben (20) über das Drehelement (30) mehr als 5 mm, vorzugsweise mehr als 8 mm und/oder weniger als 15 mm, vorzugsweise weniger als 12 mm verschiebbar ist.
und/oder dass das Drehelement (30) ein Gewinde (31) mit einer Steigung zwischen 1.5 und 7 mm aufweist
und/oder dass Hauptgehäuse (40), Kolben (20) und Drehelement (30) aus Kunststoff ausgebildet sind,
und/oder dass Kolben (20) und Drehelement (30) ein Drehlager mit einer Übermaßpassung bilden,
und/oder dass das Drehelement (30) einen Außendurchmesser von 18 bis 28 mm aufweist.

## Claims

1. An injector (1) for an intraocular lens, comprising a housing with a cannula (10), a receiving chamber (12) for the intraocular lens, and a plunger (20) which can be moved within the housing and which is adapted to move the intraocular lens from the receiving chamber (12) through the cannula (10), wherein the injector (1) comprises a rotary element (30) for moving the plunger (20);
**characterised in that** the rotary element (30) is mounted on the plunger (20) for rotation and axial translation thereon; wherein, in a starting position, the rotary element (30) is spaced apart from a seat (43) provided on a main housing (40), wherein the rotary element (30) is displaceable together with the plunger (20) until the rotary element (30) comes into contact with the seat (43) and then a thread (31) thereof engages with the seat (43).

2. The injector (1) according to the preceding claim, **characterised in that** the injector (1) is adapted so that the thread (32) engages with the seat when the intraocular lens is located inside the cannula (10), in particular when the intraocular lens is located immediately in front of a tip (11) of the cannula (10).

3. The injector (1) according to the preceding claim, **characterised in that** the intraocular lens can be pushed out of the cannula (10) by turning the rotary element (30).

4. The injector (1) according to any one of the preceding claims, **characterised in that** the rotary element (30) on the plunger (20) is secured against displacement in the distal direction by a locking ring (2).

5. The injector (1) according to any one of the preceding claims, **characterised in that**, at an end position, advancement of the plunger (20) is blocked by a resilient stopper (4).

6. The injector (1) according to any one of the preceding claims, **characterised in that** once the rotary element (30) has reached its end position, the plunger (20) can be further advanced using a handpiece (22), in particular over a distance of 1 to 8 mm.

7. The injector (1) according to any one of the preceding claims, **characterised in that** the intraocular lens is arranged in the receiving chamber (12) in a folded state; and/or **in that** the plunger (20) is latched to a main housing (40);
and/or **in that** the rotary element (30) is substantially in the form of a circular cylinder having a proximal wall that has a central opening which, in cooperation with a plunger rod (21), defines a rotary bearing;
and/or **in that** the plunger rod (21) has a cross-shaped cross section;
and/or **in that** the seat (43) for the rotary element (30) has a segmented external thread (48);
and/or **in that** the rotary element (30) engages on the seat (43) after an advancement of the plunger (20) by more than 10 mm and/or by less than 30 mm;
and/or **in that** the plunger (20) is displaceable using the rotary element (30) by more than 5 mm, preferably more than 8 mm, and/or by less than 15 mm, preferably less than 12 mm;
and/or **in that** the rotary element (30) has a thread (31) having a pitch between 1.5 and 7 mm;
and/or **in that** the main housing (40), the plunger (20), and the rotary element (30) are made of plastics material;
and/or **in that** the plunger (20) and the rotary element (30) define a rotary bearing with an interference fit;
and/or **in that** the rotary element (30) has an outer diameter between 18 and 28 mm.

## Revendications

1. Injecteur (1) pour une lentille intraoculaire, comprenant un boîtier muni d'une canule (10), une chambre de réception (12) pour la lentille intraoculaire, ainsi qu'un piston (20) déplaçable dans le boîtier, conçu pour déplacer la lentille intraoculaire hors de la chambre de réception (12) à travers la canule (10), l'injecteur (1) comprenant un élément rotatif (30) pour le déplacement du piston (20),
**caractérisé en ce que** l'élément rotatif (30) est monté de manière rotative et axialement déplaçable sur le piston (20), l'élément rotatif (30) étant, dans une position initiale, espacé d'un logement (43) disposé sur un boîtier principal (40), l'élément rotatif (30) étant déplaçable avec le piston (20) jusqu'à ce que l'élément rotatif (30) entre en contact avec le logement (43) et ensuite entre en prise avec le logement (43) au moyen d'un filetage (31).

2. Injecteur (1) selon la revendication précédente, **caractérisé en ce que** l'injecteur (1) est configuré de telle sorte que le filetage (32) entre en prise avec le logement lorsque la lentille intraoculaire se trouve dans la canule (10), en particulier lorsque la lentille intraoculaire se trouve immédiatement avant une pointe (11) de la canule (10).

3. Injecteur (1) selon la revendication précédente, **caractérisé en ce que** la lentille intraoculaire est déplaçable hors de la canule (10) par une rotation à l'élément rotatif (30).

4. Injecteur (1) selon l'une quelconque des revendications précédentes, **caractérisé en ce que** l'élément rotatif (30) sur le piston (20) est retenu par un anneau de sécurité (2) contre un déplacement en direction distale.

5. Injecteur (1) selon l'une quelconque des revendications précédentes, **caractérisé en ce que**, dans une position terminale, un avancement du piston (20) est bloqué par un butoir élastique (4).

6. Injecteur (1) selon l'une quelconque des revendications précédentes, **caractérisé en ce que** le piston (20), après que l'élément rotatif (30) a atteint la position terminale, est déplaçable davantage à l'aide d'une poignée (22), en particulier sur une distance de 1 à 8 mm.

7. Injecteur (1) selon l'une quelconque des revendications précédentes, **caractérisé en ce que** la lentille intraoculaire est disposée en état plié dans la chambre de réception (12); et/ou **en ce que** le piston (20) est encliqueté dans un boîtier principal (40);
et/ou **en ce que** l'élément rotatif (30) est essentiellement configuré comme un cylindre circulaire avec une paroi proximale, laquelle présente une ouverture centrale, qui en coopération avec une tige de piston (21) forme un palier rotatif;
et/ou **en ce que** la tige de piston (21) présente une section transversale en forme de croix;
et/ou **en ce que** le logement (43) pour l'élément rotatif (30) présente un filetage extérieur segmenté (48);
et/ou **en ce que** l'élément rotatif (30) vient en appui sur le logement (43) après un avancement du piston (20) de plus de 10 mm et/ou de moins de 30 mm;
et/ou **en ce que** le piston (20) est déplaçable par l'élément rotatif (30) de plus de 5 mm, de préférence de plus de 8 mm, et/ou de moins de 15 mm, de préférence de moins de 12 mm;
et/ou **en ce que** l'élément rotatif (30) présente un filetage (31) avec un pas compris entre 1,5 et 7 mm;
et/ou **en ce que** le boîtier principal (40), le piston (20) et l'élément rotatif (30) sont réalisés en plastique;
et/ou **en ce que** le piston (20) et l'élément rotatif (30) forment un palier rotatif avec un ajustement avec serrage;
et/ou **en ce que** l'élément rotatif (30) présente un diamètre extérieur de 18 à 28 mm.
